## Europäisches Patentamt

## European Patent Office

## Office européen des brevets

(19)

(11) Veröffentlichungsnummer : **0 056 424**
**B1**

(12) **EUROPÄISCHE PATENTSCHRIFT**

(45) Veröffentlichungstag der Patentschrift :
17.07.85

(51) Int. Cl.⁴ : **G 01 N 25/08**

(21) Anmeldenummer : 81100334.2

(22) Anmeldetag : 17.01.81

(54) Verfahren und Vorrichtung zur Bestimmung des Siedepunktes von Bremsflüssigkeit.

(43) Veröffentlichungstag der Anmeldung :
28.07.82 Patentblatt 82/30

(45) Bekanntmachung des Hinweises auf die Patenterteilung : 17.07.85 Patentblatt 85/29

(84) Benannte Vertragsstaaten :
AT CH DE FR GB IT LI

(56) Entgegenhaltungen :
DE-A- 2 655 343
DE-A- 2 658 215
DE-A- 2 721 232

(73) Patentinhaber : FAG KUGELFISCHER GEORG SCHÄFER Kommanditgesellschaft auf Aktien
Georg-Schäfer-Strasse 30
D-8720 Schweinfurt (DE)

(72) Erfinder : Peuker, Karl, Ing. grad.
Max Reger-Strasse 19
D-8603 Ebern (DE)

(74) Vertreter : Rehmann, Klaus H.
Postfach 1260 (FAG) Hauptbahnhofstrasse
D-8720 Schweinfurt (DE)

## Beschreibung

Die Erfindung betrifft ein Verfahren zur Bestimmung des Siedepunktes von Bremsflüssigkeit durch Erhitzen bis zum Siedepunkt, wobei die Bremsflüssigkeit von einem Heizelement erhitzt und die Temperatur gemessen wird, und eine Vorrichtung zur Durchführung des Verfahrens nach dem Oberbegriff des Anspruchs 3.

Es ist ein Bremsflüssigkeits-Testgerät bekannt, bei dem mittels einer Injektionsspritze Bremsflüssigkeit aus dem Bremssystem entnommen und in den Heizbecher des Testgeräts gefüllt wird. Nach dem Verschließen des Heizbechers wird die Bremsflüssigkeit erhitzt bis sich Dampfblasen bilden, die die Flüssigkeit durch ein U-Rohr in einen Auffangbehälter drücken. Dabei wird die Temperatur des Dampfblasenbildungspunktes angezeigt. Anschließend muß die Bremsflüssigkeit mit der Injektionsspritze wieder abgesaugt und das Testgerät und die Spritze wieder sorgfältig gereinigt werden. Ein solches Testgerät ist teuer und umständlich zu bedienen und daher für den Gebrauch in einer Kfz-Werkstatt nicht sehr geeignet.

Aus der DE-A- 26 55 343 ist noch ein Verfahren und eine Einrichtung zur Untersuchung des Siedepunktes von Kraftfahrzeug-Bremsflüssigkeit bekannt, bei dem der Druckanstieg detektiert wird, der auftritt, wenn der Siedepunkt der Flüssigkeit erreicht ist und sich Dampfblasen bilden. Nachteilig dabei ist, daß zwei verschiedene physikalische Größen, nämlich Druck und Temperatur meßtechnisch erfaßt werden müssen, die dann zu elektrischen Signalen umgeformt und ausgewertet werden. Der Aufwand für das Justieren und Eichen vor dem eigentlichen Meßvorgang ist relativ hoch und kann zu Fehlern führen.

Es ist deshalb Aufgabe der Erfindung, ein Verfahren und eine Vorrichtung zur Bestimmung des Siedepunktes von Bremsflüssigkeit aufzuzeigen, das einfach anzuwenden, betriebssicher und billig durchzuführen ist.

Die Lösung der Aufgabe ist aus dem kennzeichnenden Teil des Anspruchs 1 bzw. Anspruchs 3 zu entnehmen. Weitere vorteilhafte Ausführungen enthalten die abhängigen Ansprüche.

Durch das Verfahren und die Vorrichtung nach der Erfindung ist es nun möglich, die Bestimmung des Siedepunktes der Bremsflüssigkeit auch unter nich labormäßigen Bedingungen z. B. in der Werkstatt durchzuführen oder sogar im Kfz vorzunehmen, indem z. B. im Vorratsbehälter der Bremsflüssigkeit eine Meßsonde angebracht wird. Die Auswertung der Messung könnte z. B. im Rahmen der sogenannten « Check-Control » erfolgen und eine Warnleuchte aufleuchten lassen, wenn der Siedepunkt der Bremsflüssigkeit unter einem bestimmten Wert liegt.

Zur Bestimmung des Siedepunktes wird die Meßsonde z. B. in den Vorratsbehälter am Hauptbremszylinder eingetaucht. Die im Hohlraum befindliche Luft entweicht durch die Entlüftungsbohrung, die danach verschlossen wird. Nun wird der elektrische Widerstand mit Spannung versorgt und die im Hohlraum befindliche Bremsflüssigkeit erhitzt. Gleichzeitig wird die Temperatur und die zeitliche Temperaturänderung $dT/dt$ gemessen. Bei Erreichen des Siedepunktes bildet sich im oberen Bereich des Hohlraumes eine Gasblase. Da die Wärmeleitfähigkeit in Gas geringer ist als in Flüssigkeit, wird weniger Energie an die Umgebung abgegeben, so daß die Temperatur des Heizelements schneller steigt. Diese Änderung der zeitlichen Temperaturänderung $dT/dt$ läßt sich meßtechnisch eindeutig erfassen und dient als Maß für den Siedepunkt der Bremsflüssigkeit. Dabei kann die Auswertelektronik als Analoganzeige ausgeführt sein oder z. B. mittels Leuchtdioden erfolgen, die z. B. drei Bereiche anzeigt, nämlich grün für Bremsflüssigkeit mit ausreichend hohem Siedepunkt, rot für Bremsflüssigkeit mit nicht ausreichend hohem Siedepunkt und gelb für Bremsflüssigkeit mit gerade noch ausreichend hohem Siedepunkt.

Die Meßsonde kann auch in einem Zuspannelement (Radzylinder, Bremssattel) oder im Bremsschlauch eingebaut sein, da erfahrungsgemäß dort der Siedepunkt einige Grad niedriger ist als in Vorratsbehältern.

Die Erfindung soll an einem Ausführungsbeispiel näher erläutert werden.

Figur 1 zeigt schematisch einen Hauptbremszylinder mit einer im Vorratsbehälter eingetauchten Meßsonde.

Figur 2 zeigt den zeitlichen Verlauf der Temperatur während eines Meßvorgangs.

Figur 3 zeigt eine Vorrichtung gemäß der Erfindung im Schnitt vor Erreichen des Siedepunktes.

Figur 4 zeigt eine Vorrichtung gemäß der Erfindung im Schnitt nach Erreichen des Siedepunktes.

Figur 5 zeigt eine Ausbildung der Meßsonde mit einem als Entlüftungseinrichtung dienenden verschließbaren Ringkanal.

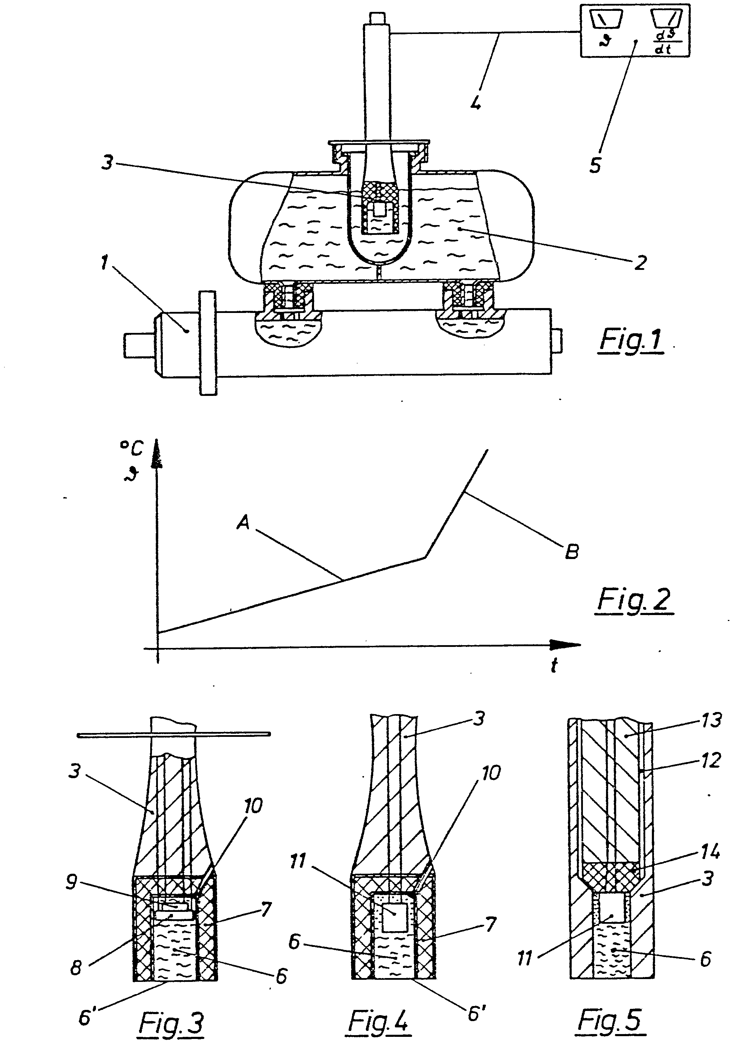

In Fig. 1 ist der Hauptbremszylinder mit 1 bezeichnet. Auf dem Hauptbremszylinder 1 ist ein Vorratsbehälter 2 angebracht, in dem eine Meßsonde 3 angeordnet ist, die über ein Kabel 4 mit einer Anzeige- und Auswertelektronik 5 verbunden ist. Wie aus Fig. 2 ersichtlich, steigt nach Anlegen der Heizspannung die Temperatur des Heizelements 8 im Bereich A stetig an. Bei Erreichen des Siedepunktes der Bremsflüssigkeit ändert sich der Temperaturverlauf und geht in den Bereich B über. Diese Änderung wird von der Auswertelektronik registriert und die Temperatur, bei der die Änderung erfolgt, angezeigt. Diese Anzeige dient nun als Maß für den Siedepunkt der Bremsflüssigkeit.

Zu bemerken ist noch, daß das erfindungsgemäße Verfahren und die Vorrichtung auch dazu verwendet werden können, den Füllstand der Bremsflüssigkeit im Vorratsbehälter zu kontrollieren und ein Warnsignal auszulösen, wenn der Flüssigkeitsstand unter einen bestimmten Wert (= Eintauchhöhe der Meßsonde) sinkt. Dazu muß in der Auswertelektronik lediglich ein Vergleich der Temperaturverläufe durchgeführt werden. Verläuft der Temperaturanstieg ähnlich dem Bereich A ist die Meßsonde noch in die Bremsflüssigkeit eingetaucht. Verläuft der Temperaturanstieg jedoch ähnlich dem Bereich B, ist die Meßsonde nicht mehr eingetaucht und es muß Bremsflüssigkeit nachgefüllt werden.

In Fig. 3 ist die eingetauchte Meßsonde dargestellt. Der Hohlraum 6 ist mit Bremsflüssigkeit, die durch die Öffnung 6' eingedrungen ist, gefüllt und von einer Isolierschicht 7 umgeben. Im oberen Bereich des Hohlraumes 6 ist ein Heizelement 8 und ein Temperaturfühler 9 angeordnet. Über eine verschließbare Entlüftungsbohrung 10 kann etwa eingeschlossene Luft entweichen.

Wie aus Fig. 4 ersichtlich, können der Temperaturfühler und das Heizelement auch zu einer Einheit zusammengefaßt sein, indem z. B. ein Kaltleiter-Widerstand 11 verwendet wird, der sowohl heizt, als auch seinen Widerstand temperaturabhängig ändert.

In Fig. 5 ist eine Meßsondenausführung gezeigt, bei der ein Ringkanal 12 zur Entlüftung dient. Dazu ist die Meßsonde 3 mehrteilig ausgeführt, wobei in dem rohrförmigen Teil der Meßsonde 3 ein axial verschiebbares Teil 13 mit einer am Ende angebrachten Dichtung 14 angeordnet ist. Das Teil 13 besitzt einen geringeren Durchmesser als die Bohrung des rohrförmigen Teils. Dadurch entsteht ein Ringraum 12, durch den die Luft aus dem Hohlraum 6 entweichen kann, wenn die Dichtung 14 angehoben ist.

Die Auswertung der gemessenen Temperaturen und Temperaturänderungen erfolgt in einer Anzeige- und Auswertelektronik 5, die z. B. über Kabel mit der Meßsonde 3 verbunden ist. Die Auswertelektronik kann auch in die Meßsonde integriert sein oder z. B. im Deckel des Vorratsbehälters angeordnet sein, wo z. B. über farbige Leuchtdioden der Zustand der Bremsflüssigkeit nach folgenden Kriterien beurteilt wird :

Bremsflüssigkeit in Ordnung (grün)

Bremsflüssigkeit gerade noch in Ordnung sollte aber bald ausgetauscht werden (gelb)

Bremsflüssigkeit nicht in Ordnung, sofort austauschen (rot).

## Patentansprüche

1. Verfahren zur Bestimmung des Siedepunktes von Bremsflüssigkeit durch Erhitzen bis zum Siedepunkt, wobei die Bremsflüssigkeit von einem Heizelement (8, 11) erhitzt und die Temperatur gemessen wird, dadurch gekennzeichnet, daß auch die zeitliche Temperaturänderung des Heizelements (8, 11) gemessen wird und daß bei einer Änderung der zeitlichen Temperaturänderung die gerade erreichte Temperatur registriert wird und als Maß für den Siedepunkt der Bremsflüssigkeit dient.

2. Verfahren nach Anspruch 1, dadurch gekennzeichnet, daß die Bestimmung der Temperatur und der zeitlichen Temperaturänderung mittels elektrischer Widerstandsmessung erfolgt und die Stromstärke ein Maß für die Temperatur ist, wobei der elektrische Widerstand das Heizelement (8, 11) ist, das vorzugsweise als Kalt- oder Heißleiter-Widerstand ausgeführt ist.

3. Vorrichtung zur Durchführung des Verfahrens nach Anspruch 1, bei der in einer in die Bremsflüssigkeit getauchten Meßsonde (3) ein Hohlraum (6) vorgesehen ist, in dem ein Heizelement (8) und ein Temperaturfühler (9) angeordnet sind, bei der die von dem Temperaturfühler (9) ermittelten Werte einer Anzeige- und Auswertelektronik (5) zugeführt werden, und bei der der untere Bereich des Holraums (6) mit mindestens einer Öffnung (6') zum Eindringen der Bremsflüssigkeit versehen ist, dadurch gekennzeichnet, daß die Anzeige- und Auswertelektronik (5) derart ausgebildet ist, daß sie die Temperatur anzeigt, bei der eine Änderung der zeitlichen Temperaturänderung beim Aufheizen der im Hohlraum (6) befindlichen Bremsflüssigkeit erfolgt und daß der obere Bereich des Hohlraums (6) gasdicht ausgebildet ist und das Heizelement (8), den Temperaturfühler (9) und eine absperrbare Entlüftungseinrichtung (10 ; 12) aufweist.

4. Vorrichtung nach Anspruch 3, dadurch gekennzeichnet, daß Heizelement (8) und Temperaturfühler (9) zusammengefaßt sind.

5. Vorrichtung nach Anspruch 4, dadurch gekennzeichnet, daß das Heizelement (8) und der Temperaturfühler (9) als ein elektrischer Widerstand (11), vorzugsweise als Kalt- oder Heißleiterwiderstand, ausgebildet sind.

6. Vorrichtung nach Anspruch 3, dadurch gekennzeichnet, daß die Entlüftungseinrichtung als eine Entlüftungsbohrung (10) oder ein Ringkanal (12) ausgeführt ist.

7. Vorrichtung nach Anspruch 3, dadurch gekennzeichnet, daß der Hohlraum (6) von einer Isolierschicht (7) umgeben ist.

8. Vorrichtung nach Anspruch 3, dadurch gekennzeichnet, daß die Meßsonde (3) ganz oder teilweise aus Material mit geringer Wärmeleitung, z. B. Kunststoff, hergestellt ist.

9. Vorrichtung nach Anspruch 3, dadurch gekennzeichnet, daß die Meßsonde (3) mit einer integrierten Analog- oder Leuchtdiodenanzeige versehen ist.

10. Vorrichtung nach Anspruch 3, dadurch gekennzeichnet, daß die Meßsonde (3) in den Deckel des Ausgleichsbehälters integriert ist.

## Claims

1. Method for determination of the boiling point of brake fluid by heating it up to the boiling point with the aid of a heater (8, 11) and the temperature being measured, characterized by the possibility of measuring also the temperature change as a function of time and recording the temperature change occuring in the moment when the brake fluid boiling point is achieved.

2. Method according to claim 1, characterized by the determination of the temperature and the temperature change as a function of time being made with the aid of electrical resistors, the amperage being the unit of temperature, the electrical resistor being the heater (8, 11) which can be designed as cold or hot thermistor.

3. Device to carry through the method according to claim 1 in which a probe (3) with a cylindrical chamber (6) is immersed into the brake fluid. The cylindrical chamber (6) is equipped with a heater (8) and a sensor (9) which passes on the values received to an electronic evaluation system (5). At the bottom of the cylindrical chamber (6) is at least one opening (6') for the brake fluid to enter into the chamber, characterized by the display and evaluation system (5) being designed so that the change of temperature rise as function of time during heating up of the brake fluid in the cylindrical chamber (6) is indicated. The upper part of the cylindrical chamber (6) is gastight and features the heater (8), a sensor (9) and a vent (10, 12) which can be closed.

4. Device according to claim 3, characterized by the heater (8) and the sensor (9) being connected.

5. Device according to claim 4, characterized by the heater (8) and sensor (9) being designed as electrical resistor (11) preferably as cold or hot thermistor.

6. Device according to claim 3, characterized by the vent being designed either as bore (10) or as annular duct (12).

7. Device according to claim 3, characterized by the cylindrical chamber (6) being surrounded by an isolating layer (7).

8. Device according to claim 3, characterized by the probe (3) being completely or partly made of material with low thermal conductivity, e. g. synthetic material.

9. Device according to claim 3, characterized by the probe (3) being equipped with an integral analog of LED display.

10. Device according to claim 3, characterized by the probe (3) being made integral with the cover of the reservoir.

## Revendications

1. Procédé pour la détermination du point d'ébullition de liquide de frein par chauffage jusqu'au point d'ébullition où le liquide de frein est chauffé par un élément de chauffage (8, 11) et la température est mesurée et caractérisé en ce que le changement de température dans le temps de l'élément de chauffage (8, 11) est également mesuré, et où au moment d'un changement de la courbe de température, on enregistre la température alors atteinte, celle-ci étant caractéristique pour le point d'ébullition du liquide de frein.

2. Procédé selon la revendication 1, caractérisé en ce que la détermination de la température et le changement de température dans le temps sont mesurés par résistance électrique, et que l'intensité du courant sert de mesure pour la température ; la résistance électrique est l'élément de chauffage (8, 11) qui est de préférence conçue en tant que thermistor positif ou négatif.

3. L'appareil pour la mise en œuvre du procédé selon revendication 1, comprend une sonde de mesure (3) immergée dans le liquide de frein présentant une chambre (6) où sont disposés un élément de chauffage (8) et une sonde de température (9) et dont les valeurs déterminées par la sonde de température (9) sont transmises à une électronique d'affichage et d'évaluation (5). La partie inférieure de la chambre (6) est au moins pourvue d'une ouverture (6) servant à la pénétration du liquide de frein, et caractérisé en ce que l'électronique d'évaluation et d'affichage (5) est conçue pour l'enregistrement de la température à laquelle intervient un brusque changement de la température lors du chauffage du liquide de frein se trouvant dans la chambre (6) et que la partie supérieure de la chambre (6) est étanche au gaz et contient un élément de chauffage (8), une sonde de mesure (9) et un dispositif de désaérage (10, 12) pouvant être obturé.

4. Appareil selon revendication 3, caractérisé en ce que l'élément de chauffage (8) et la sonde de température (9) sont regroupés.

5. Appareil selon revendication 4, caractérisé en ce que l'élément de chauffage (8) et la sonde de température (9) sont conçus en tant que résistance électrique (11), de préférence en tant que thermistor positif ou négatif.

6. Appareil selon la revendication 3, caractérisé en ce que le dispositif de ventilation est conçu en tant que trou de désaérage (10) ou canal annulaire (12).

7. Appareil selon revendication 3, caractérisé en ce que la chambre (6) est protégée par une gaine isolante (7).

8. Appareil selon revendication 3, caractérisé en ce que la sonde de mesure (3) est complètement ou en partie fabriquée à base de matériau dont la conduction de chaleur est faible, p. ex. en matière synthétique.

9. Appareil selon revendication 3, caractérisé en ce que la sonde de mesure (3) est équipée d'un affichage ou de diodes lumineuses intégrés.

10. Appareil selon revendication 3, caractérisé en ce que la sonde de mesure (3) est intégrée dans le couvercle du réservoir de compensation.

*Fig.1*

*Fig.2*

*Fig.3*

*Fig.4*

*Fig.5*